# EUROPEAN PATENT SPECIFICATION

(11) **EP 2 628 394 B1**
(45) Date of publication and mention of the grant of the patent: **06.04.2016**
(21) Application number: 11831903.7
(22) Date of filing: 14.10.2011
(51) Int. Cl.: A23L 33/00, A23G 9/36, A61K 35/747

(54) **PROBIOTIC FUNCTIONAL FOOD SUITABLE FOR IMMUNOCOMPROMISED INDIVIDUALS UNDERGOING TREATMENT SUCH AS CHEMOTHERAPY AND/OR RADIOTHERAPY**
PROBIOTISCHES FUNKTIONALES NAHRUNGSMITTEL FÜR DURCH EINE BEHANDLUNG WIE EINE CHEMOTHERAPIE UND/ODER EINE RADIOTHERAPIE IMMUNGESCHWÄCHTE PATIENTEN
ALIMENT FONCTIONNEL PROBIOTIQUE CONVENANT POUR LES INDIVIDUS IMMUNODÉPRIMÉS RECEVANT DES TRAITEMENTS TELS QUE CHIMIOTHÉRAPIE ET OU RADIOTHÉRAPIE

(30) Priority: 14.10.2010 CL 11242010
(43) Date of publication of application: 21.08.2013
(73) Proprietor: Universidad De Concepcion, Concepción (CL); Ormeño Saavedra, María Loreto, Concepción (CL)
(72) Inventor: ORMEÑO SAAVEDRA, María, Loreto, Concepción (CL); CASTRO INOSTROZA, Erica, Concepción (CL); BORQUEZ YAÑEZ, Rodrigo, Concepción (CL); GONZALEZ RIQUELME, Margarita, Concepción (CL); VERA GARCIA, Rodrigo, Concepción (CL); TOLEDO AGUILAR, Natalia, Concepción (CL)
(74) Representative: Carvajal y Urquijo, Isabel
(86) International application number: PCT/CL2011/000064
(87) International publication number: WO 2012/048438

(56) References cited:
- WO-A1-2006/054135
- RU-C2- 2 208 942
- US-A1- 2004 057 943
- GIRALT J ET AL: "Effects of Probiotic Lactobacillus Casei DN-114 001 in Prevention of Radiation-Induced Diarrhea: Results From Multicenter, Randomized, Placebo-Controlled Nutritional Trial", INTERNATIONAL JOURNAL OF RADIATION: ONCOLOGY BIOLOGY PHYSICS, PERGAMON PRESS, USA, vol. 71, no. 4, 15 July 2008 (2008-07-15), pages 1213-1219, XP022760845, ISSN: 0360-3016, DOI: 10.1016/J.IJROBP.2007.11.009 [retrieved on 2008-02-19]
- HEKMATS ET AL: "Survival of Lactobacillus acidophilus and Bifidobacterium bifidum in ice cream for use as a probiotic food", JOURNAL OF DAIRY SCIENCE, AMERICAN DAIRY SCIENCE ASSOCIATION, US, vol. 75, no. 6, 1 January 1992 (1992-01-01), pages 1415-1422, XP002136021, ISSN: 0022-0302, DOI: 10.3168/JDS.S0022-0302(92)77895-3
- EL-ATTI S.A. ET AL.: 'Use of probiotics in the management of chemotherapy-induced diarrhea: a case study' JOURNAL OF PARENTERAL AND ENTERAL NUTRITION vol. 33, no. 5, September 2009 - October 2009, pages 569 - 570, XP009172162
- DEL PIANO M. ET AL.: 'Evaluation of the intestinal colonization by microencapsulated probiotic bacteria in comparison with the same uncoated strains' J. CLIN. GASTROENTEROL. vol. 44, no. SUPPL.1, September 2010, pages S42 - S46, XP009159527

## Description

### STATE OF THE ART

Over the last few years, interest in preventive therapies and nutritional supplements to improve health has been increasing. The fact that human beings are genetically predisposed to acquire certain diseases has led researchers to look for new alternatives to prevent these diseases, among which diet is one of the most important factors. According to the American Cancer Society, 1.5 million new cancer cases are registered each year and it is estimated that 70% of these cases are due to diet. The devastating effects of this disease can be prevented or attenuated by a balanced diet, with a consequent change in eating habits.

In Chile, every year about 40,000 individuals develop some type of cancer. However, a high percentage of cancer sufferers in the world now live with the disease or have recovered from it through surgery and follow-up treatment with chemotherapy and/or radiotherapy.

The main goal of this type of treatment is to cure the disease, but some types of cancer cannot be cured, in which case therapies are used to improve the quality of life of the patient in some way.

The drugs used in chemotherapy do not only affect cancer cells but also normal cells and they generally have undesirable side effects.

Nausea, vomiting, fatigue, hair loss and severe stomach problems, in addition to symptoms that are imperceptible to the patient, such as a fall in red and white blood cell counts and blood platelets, are some of the side effects of chemotherapy about which patients frequently complain.

Most of these effects disappear when treatment ends, enabling healthy cells to stabilize their metabolism and reproduce to reach their normal number. In the meantime, there are various ways in which patients can control particular minor problems. In the case of severe side effects, the doctor makes certain recommendations to palliate or relieve unpleasant reactions to some extent. In general, people tend to feel better during cancer treatment if they rest enough and have a balanced diet. Many patients need to sleep more, as well as to ingest more proteins and calories, to help the body to recover. Chemotherapy frequently causes constipation or diarrhea. In addition, the drugs used in this type of treatment reduce saliva production, causing a dry mouth with the risk of lesions and ulceration, known medically as mucositis. The resulting different levels of infection can be very painful for patients, preventing them from eating properly. To reduce this risk, it is essential to keep the mouth clean and moist by drinking lots of fluids and consuming food that is refreshing to the palate with a high nutritional content, as cancer therapy leads to a great loss of proteins, carbohydrates, and vitamins, etc., which is demonstrated in the reduction in patients' muscular mass and considerable weight loss.

Taking into account all the afore-mentioned, the proposed initiative significantly contributes to counteracting the negative effects of chemotherapy.

### CURRENT STATE OF THE ART

The use of probiotics in the treatment of diseases, such as in cancer patients undergoing therapy, has been disclosed in US 2004/057943; Giralt et al, Int. J. Radiatio Oncology Biol. Phys. 2008; 71 (4): 1213-1219, and in EI-Atti et al, JPEN 2009; 33 (5): 569-570. Moreover, a search was made in the main invention patent offices in the world and the following documents relevant to the present invention were found:

The US invention patent US5922375 (1999) Probiotic bifidobacterium strain, describes a probiotic bifidobacterium strain isolate that is incorporated into liquid foods, animal feeds, and/or dietary supplements. It is given as a healthy bacterium to humans and other mammals. This bacterium helps newborns to produce acetic acid and protective lactic acid, as well as antimicrobials and vitamins. The bacterium can also be used to reseed bacteria levels lost through diarrhea, chemotherapy, advancing age, antibiotics, or other causes. The main difference between this technology and the one seeking protection lies in the strain that is being claimed and its use, in addition to the fact that protection is aimed at the incorporation of food.

At the world patent office, invention patent WO0182711 (2001), Lactic acid food *products,* refers to food products that contain the probiotic strain *Lactobacillus acidophilus* and soy milk. The foods mentioned include a variety of dairy products such as sour cream, cottage cheese, desserts and ice cream. The difference between this technology and the initiative seeking protection lies mainly in the formulation used for the preparation of the said foodstuffs and the use given to the formulation.

Chinese invention patent CN1552229 (2004), Health-care ice cream powder containing active lactic acid bacteria, protects an ice cream dessert to safeguard health that is in powder form and contains active lactobacilli, prepared from sugar, powdered milk, vegetable butter, maltodextrine, sucrose ester, gum, CMC, essence, and coloring, etc. This product can maintain an ecological microenvironment in the human body and promotes the decomposition and absorption of nutrients. It is different from the technology seeking protection with regard to the formulation and use given to this functional food.

The Swedish invention patent SE526711 (2005), New strain of bifidobacterium with the ability to survive in the intestinal tract and produce glutamine and arginine in vivo, useful for preparing medicine for the treatment of intensive care patients with intestinal failure, protects a bifidobacterium strain with the ability to survive in the intestinal tract and produce glutamine *in vivo.* This patent claims a bifidobacterium strain, a composition that includes the strain with a carrier and a medicine for the treatment of patients with multiorgan dysfunction and intestinal damage requiring intensive care. This medicine can be used for prophylaxis in patients on chemotherapy and for patients with inflammatory or post-operative problems. The initiative seeking protection is not a medicine and the strain used is different from the one claimed in the above invention patent.

The Russian invention patent RU2294647 (2007), Ice cream with functional characteristics, protects an ice cream consisting of milk, cream, sugar, stabilizers, vitamin complexes and a bacteria concentrate containing *Bifidobacterium longum, Lactobacillus acidophilus* and *Propionibacterium shermanii.* The technology seeking protection is different from the above, both in its formulation and in the use given to the formulation.

A similar document is RU 2 208 942. In addition to the review of existing patents, a search was conducted for relevant documents or publications relating in any way to the technology seeking protection. Among the documents found there are a variety of publications that mention probiotic foods, such as: **Survival of lactobacillus acidophilus and bifidobacterium bifidum in ice cream for use as a probiotic food** (Sharareh Hekmat, 1992. Journal of Dairy Science 75: 1415-1422) and **Survival of probiotic microoganisms lactobacillus acidophilus and bifidobacterium lactis in whipped ice cream** (Alejandro Corrales, June 2007. Revista Chilena de Nutrición Vol. 34 N° 2, pp. 157-163). These articles compare two probiotic strains, measuring parameters such as survival, changes in the organoleptic properties of the product, etc. Other publications were also found relating to the utility of certain foods for people suffering from colitis or immunological problems, but none of these foods totally reproduces the initiative proposed here.

### DESCRIPTION OF THE INVENTION

The present invention is defined by the claims and broadly corresponds to a functional food for cancer patients who are immunocompromised because of the treatment they have been given. This food is prepared from a probiotic formulation whose active principle includes viable probiotic strains that produce lactic acid, *Lactobacillus* spp, preferably but not exclusively, the *Lactobacillus plantarum* strain DSM 22105, isolated from breast milk, conditioned to withstand temperatures between -30 and -40°C, maintaining its concentration in a range from 10⁶ - 10⁹ CFU/ml, for long periods of time of at least 120 days. This formulation makes possible the production of functional foods, preferably ice cream, in addition to jellies, desserts, juices and/or milk derivatives, which are administered orally to cancer patients, preferably on chemotherapy and radiotherapy treatment, where the said food favors weight increase in these patients and counteracts the side effects produced by these types of therapies, such as mucositis, mouth dryness, lesions and erosions in the digestive tract.

To create this product, first the DSM 22105, isolated from breast milk, was selected from a number of probiotic strains, because it is the microorganism that has shown the most promising results in comparison with other strains of the same origin. (Table **1)**

**Table 1**

| **Strains** | **Origin** | **Hydrophobicity (%)** | | | **Peroxide Production** |
|---|---|---|---|---|---|
| | | **Hexadecane** | **Toluene** | **Xylene** | |
| **LPM O1** *L. **plantarum*** | Breast milk | 85 | 65.9 | 64.7 | + |
| **LPM B₄** *L. **plantarum*** | Breast milk | 74 | 80.6 | 69.9 | +++ |
| **LPM AK1** *L. **plantarum*** | Breast milk | 80.6 | 86.7 | 79.2 | +++ |
| **LPM AR2 *Lactobacillus* spp.** | Breast milk | 84 | 88 | 91 | - |
| **LPM P1** *L. **salivarius*** | Breast milk | 66.5 | 72.6 | 68.2 | ++ |

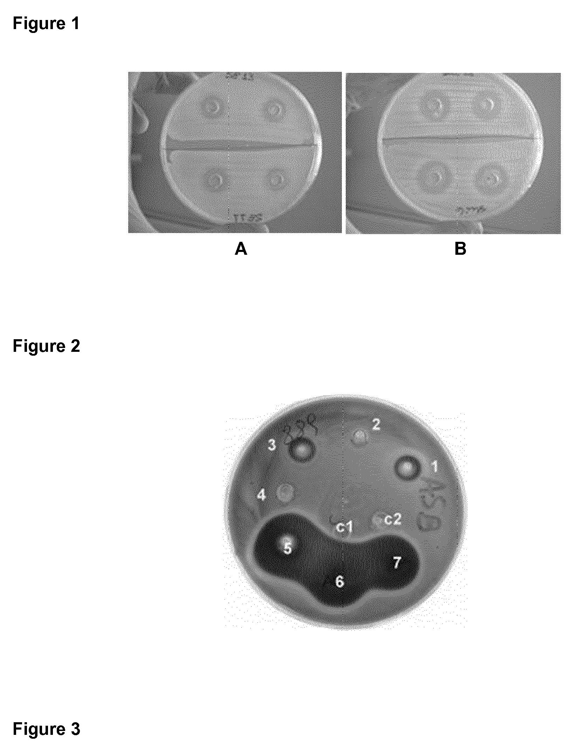

Studies have shown that, in the case of gastrointestinal pathologies, probiotic bacteria can be used both for therapeutic and preventive purposes. If these bacteria are added to certain foods in different ways, they are effective in the prevention and treatment of various illnesses. Some studies show that children given probiotic supplements present an increase in immunoglobulins, which decreases the intensity and duration of diarrhea produced by rotavirus. Probiotics are even able to inhibit the growth of hospital pathogenic bacteria, for which reason inhibition tests were carried out, **(****Figure 1****, A** *Salmonella enteritidis,* **B** *Shigella* spp) in which the ability of strain LPM O1 (DSM 22105) isolated from breast milk to inhibit hospital pathogens and *Campylobacter jejuni* was evaluated **(****Figure 2****)** in interaction trials in a cell culture dish.

In addition to the hospital pathogen inhibition tests, immunization tests with ovalbumin (OVA) were carried out on mice fed on the DSM 22105 strain, to demonstrate the beneficial effects of this strain To this purpose, the mice were organized into three experimental groups and two control groups, in the following way:
*Group 1 (preventive):* the mice were fed for 7 days with the DSM 22105 strain with a dose of 10⁷ in serum glucose and then they were immunized with OVA antigen.
*Group 2 (preventive):* the mice were fed for 7 days with the DSM 22105 strain with a dose of 10⁷ in serum glucose, then they were immunocompromised with dexamethasone (4 doses, one every two days) and were immunized with OVA antigen from day 7.
*Group 3 (control):* the mice were exposed to OVA antigen.
*Group 4 (control):* the mice were immunocompromised with dexamethasone and immunized with OVA antigen.
*Group 5 (curative):* the mice were immunocompromised with dexamethasone, immunized with OVA antigen, and then fed for 7 days on the DSM 22105 strain with a dose of 10⁷ in serum glucose.

Subsequently, blood samples were taken from each experimental group at 0, 7, 14, 21, and 28 days from the first immunization, and then at 7, 14, 21 and 28 days after the second immunization. **Table 2** shows the title of antibodies obtained.

**Table 2**

| Time (days) | Group 1 | Group 2 | Group 3 | Group 4 | Group 5 |
|---|---|---|---|---|---|
| 0 | - | - | - | - | - |
| 7 | - | - | - | - | - |
| 14 | 1/300 | 1/600 | 1/300 | 1/300 | 1/1000 |
| 21 | 1/600 | 1/600 | 1/600 | 1/600 | 1/1000 |
| 28 | 1/600 | 1/600 | 1/600 | 1/800 | 1/1000 |
| 7 (2°inm) | 1/6000 | **1/12000** | 1/6000 | 1/6000 | **1/12000** |
| 14 (2°inm) | 1/6000 | **1/12000** | 1/6000 | 1/6000 | **1/12000** |
| 21 (2°inm) | 1/6000 | **1/12000** | 1/6000 | 1/6000 | **1/12000** |
| 28 (2°inm) | 1/3000 | **1/6000** | 1/3000 | 1/6000 | **1/6000** |

As can be observed in **Table 2,** the results obtained by administering the DSM 22105 strain orally show that at 14 days all groups presented an increase in the title of antibodies, but the curative group presented the greatest increase. The response was similar at the different times studied. Nevertheless, after the second immunization, a much more significant antibody title increase was observed, especially in the preventive groups (immunocompromised) and the curative group, which had the highest title of antibodies, revealing that the DSM 22105 strain significantly reactivates the humoral immune response.

Once these properties have been determined, the DSM 22105 strain is conditioned and its ability to withstand extreme temperatures increased in order to finally produce a functional food, preferably a specially prepared ice cream.

To condition the strains to withstand low temperatures, certain procedures were carried out and elements produced that allow the DSM 22105 strain to acquire this property during the culture process and subsequent handling.

First, the culture of this lactic bacterium was optimized. To this purpose, a culture medium was used containing hydrolyzed whey permeate, which was the main carbon source of the medium and which, in this particular case, acted as a cryoprotectant, with additives and magnesium and manganese salts.

The addition of nonionic surfactants derived from sorbitan, such as polyoxyethylene (20) sorbitan monooleate, modified the permeability of the bacteria cell membrane, contributing to its survival during the frozen stage, because this increased the proportion of unsaturated fatty acids in the cell membrane. This fact is important as the strain in question was cultured in a medium containing polyoxyethylene (20) sorbitan monooleate and the biomass obtained was not washed, so that it contained a significant quantity of undigested nutrients, among which was the compound described above.

Another important factor is the physiological state of the bacteria. Bacteria in the stationary phase are more resistant to freezing or drying by sublimation (liophylization) than bacteria in the exponential phase. The culture was therefore harvested at 12 hours, which corresponded to the stationary phase of the DSM 22105 strain.

The addition of cryoprotectant agents, in addition to whey permeate, sorbitol, monosodium glutamate, and glycerol, among others, attenuates damage to the membrane during the freezing of the bacterial strains. At the same time, sucrose, a main component of ice cream, also has a protecting effect through its interaction with membrane proteins, thus reducing cell damage.

The nature of the additives is critical during the storage phase, since they directly influence the viability of the bacteria, but at the same time they should not alter the quality of the food product. To conclude, both the culture medium and the additives in the probiotic ice cream formulation contain cryoprotectant agents, which explains the maintenance of the viability of this product during storage time.

Once the conditions in which the DSM 22105 strain maintains its properties at low temperatures had been determined, a consignment of specially prepared ice cream was produced. This ice cream kept its organoleptic properties and possessed excellent stability indices for probiotic mass, attaining 1x10⁸ CFU/ml at the end of the monitoring process. This was determined from the samples taken from each container of prepared ice cream, all of which were homogenized and planted in different culture media to evaluate the viability (CFU/ml) and probiotic properties. Figure 3 shows that the DSM 22105 strain incorporated into the special-purpose ice cream maintained high probiotic concentrations of around 1x10⁸ CFU/ml for at least 120 days from the day of preparation.

With regard to the biochemical characteristics of the DSM 22105 strain, in Table 3 it can be observed that these characteristics were maintained with respect to the original strain, which shows that the conditioning is effective and that the strain is able to withstand low temperatures without losing its properties.

**Table 3**

| **Date** | **Arginine** | **Esculin** | **Nitrate** | **Indole** | **Gluconate** | **Gibson** | **T° 15°C** | **T° 45°C** | **Peroxide production** |
|---|---|---|---|---|---|---|---|---|---|
| *Initial* | Negative | Positive | Negative | Negative | Negative | Negative | Negative | Positive | Negative |
| *30 days* | Negative | Positive | Negative | Negative | Negative | Negative | Negative | Positive | Negative |
| 60 *days* | Negative | Positive | Negative | Negative | Negative | Negative | Negative | Positive | Negative |
| 78 *days* | Negative | Positive | Negative | Negative | Negativee | Negative | Negative | Positive | Negative |

**Table 4** shows the evolution of the probiotic property of hydrophobicity of the strain in the ice cream. It can be seen that the property was maintained with respect to the original.

**Table 4**

| **TIME** | **Index** | **XYLENE** | **TOLUENE** | **HEXADECANE** |
|---|---|---|---|---|
| *Initial* | **High** | 85.6% | 80.9% | 83.6% |
| *30 days* | **High** | 86.8% | 85.3% | 78.2% |
| *60 days* | **High** | 87.1% | 79.8% | 81.1% |
| *78 days* | **High** | 84.7% | 86.8% | 85.4% |

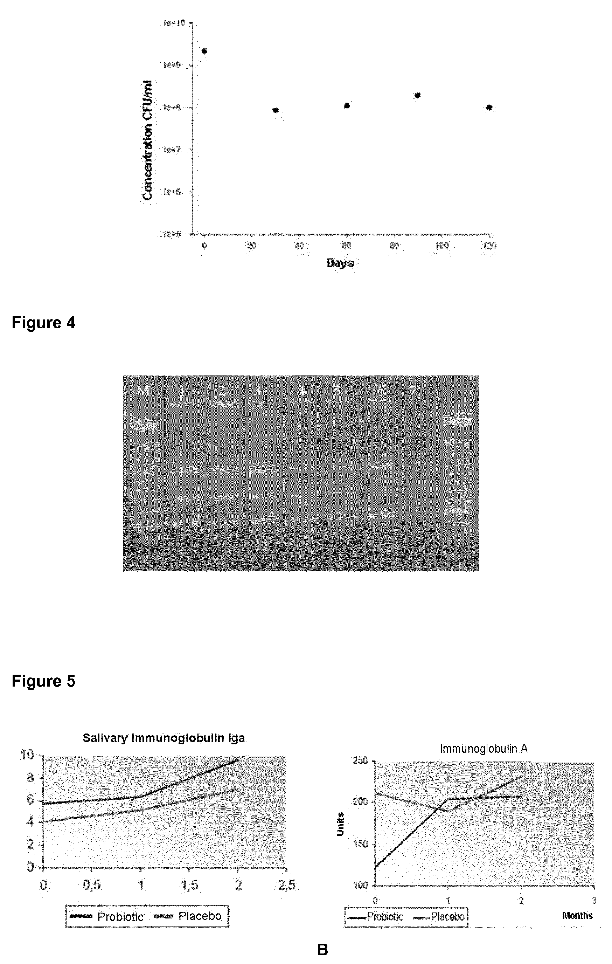

**Figure 4** shows the polymorphic profile obtained by RAPD-PCR of the DSM 22105 strain and the colonies isolated from the ice cream samples analyzed at 30 and 70 days: track 1 corresponds to the DSM 22105 strain (extraction kit); track 2 to ice cream colony at 30 days (extraction kit); track 3 to ice cream colony at 70 days (extraction kit); track 4 to the DSM 22105 strain (enzymatic extraction); track 5 to ice cream colony at 30 days (enzymatic extraction); track 6 to ice cream colony at 70 days (enzymatic extraction); track 7 to negative control and molecular weight (M) marker. In this figure it can be observed that the profiles show the same banding, the difference in intensity being produced by the type of extraction carried out, as bands 1, 2, and 3 were produced with a commercial extraction kit and bands 4, 5 and 6 were produced through enzymatic extraction.

From these analyses it can be concluded that the probiotic strain DSM 22105 incorporated into the specially prepared ice cream maintained the probiotic concentrations throughout the monitoring process. The biochemical tests on the strains isolated from the ice cream containers maintained the same profile as the incorporated probiotic strain. In addition, it can be observed that the probiotic properties of the strains isolated from the preparations were maintained with respect to the original, and the organoleptic properties of the ice cream - color, flavor and texture - were maintained without variation during the whole monitoring process.

Once these parameters had been measured, tests were conducted on patients on chemotherapy, this being the objective of the production of probiotic ice cream. Five voluntary cancer patients, with colon cancer, breast cancer and ovarian cancer, among others, all on chemotherapy, were recruited in addition to six healthy volunteers. A randomized double blind trial was designed, in which, by drawing lots, the volunteers selected the preparation according to its color, orange or green. The orange preparations contained only ice cream and the green preparations contained the probiotic strain. This identification was carried out at the moment of analyzing the results, not before, and the contents of the preparations were known only to the person in charge of the production process. The trial lasted 60 days, sensitization procedures being conducted beforehand, so that volunteers began the process with informed consent.

In the case of the healthy volunteers, a summary of the individual evolution of the participants with respect to saliva and serum immunoglobulin A is presented in **Tables 5** and **6,** as averages 3 volunteers with ice cream containing probiotics and 3 volunteers with a placebo, at 0, 30 and 60 days of ice cream consumption, and in **Figures 5** **A** and **B.** Although the results obtained are within normal ranges, a more marked rise in serum IgA levels was observed in patients who consumed probiotic ice cream than in those who consumed placebos.

**Table 5**

| Serum Immunoglobulin A (Average) | | | |
|---|---|---|---|
| | 0 | 30 days | 60 days |
| Probiotic | 122 | 204 | 208 |
| Placebo | 211 | 190 | 232 |

**Table 6**

| Salivary IgA (Average) | 0 | 30 days | 60 days |
|---|---|---|---|
| Probiotic | 5.7 | 6.3 | 9.6 |
| Placebo | 4.1 | 5.1 | 7.0 |

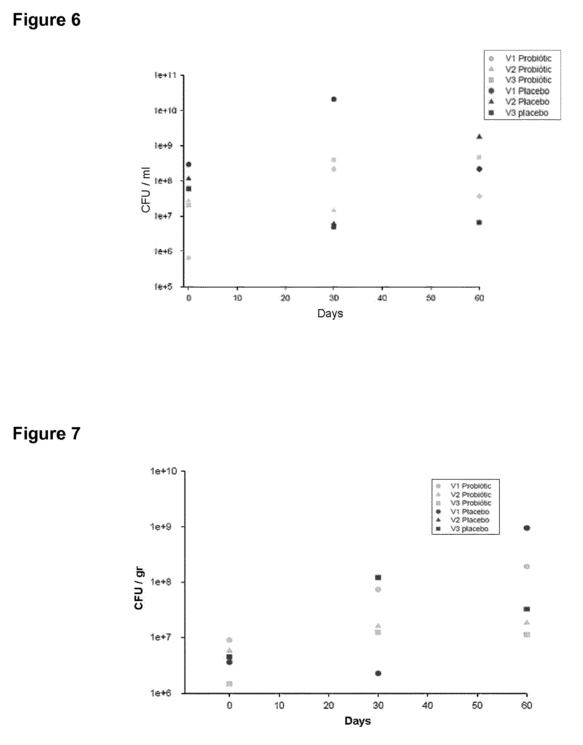

Then a microbiological recount was conducted in stools from healthy volunteers. **Figure 6** gives a graphic presentation of the lactic acid flora concentrations obtained from healthy volunteers analyzed up to 60 days. It can be observed that these volunteers had a basal lactobacillus concentration of approximately 10⁷ - 10⁹ CFU/ml. At 30 days differences were observed between the two groups of volunteers. In the probiotic group all the volunteers increased their concentration significantly, while the placebo group showed a decrease. At the end of the 60-day trial, the probiotic group presented recounts similar to those obtained at 30 days, while the placebo group showed an increased concentration in comparison with the 30-day recounts.

In the cancer volunteers, it was observed that the IgA parameters were more stable in the patients who consumed the preparation with probiotics in comparison with the patients who took the placebo **(Table 7,** as averages 3 volunteers with probiotic ice cream and 2 volunteers with the placebo, at 0, 30 and 60 days of ice cream consumption). A progressive, sustained increase over time was observed in the people on probiotic ice cream, in contrast to the volunteers on the placebo, whose evolution was more erratic. Although an increase in salivary IgA was observed at day 60, this increase was not statistically significant (ANOVA p = 0.790123).

**Table 7**

| Salivary Immunoglobulin IgA (Average) | | | |
|---|---|---|---|
| | 0 | 30 days | 60 days |
| Probiotic | 4.5 | 6.7 | 8.3 |
| Placebo | 5.4 | 3.4 | 1.7 |

With respect to IgA evolution, **(Table 8,** as averages 3 volunteers on probiotic ice cream and 2 volunteers on placebo, at 0, 30 and 60 days of ice cream consumption), behavior was very erratic in placebo users, in contrast to probiotic ice cream users, whose evolution was more stable. Although a difference was observed, this difference is not statistically significant (ANOVA p = 0.149651).

**Table 8**

| Serum Immunoglobulin A (Average) | | | |
|---|---|---|---|
| | 0 | 30 days | 60 days |
| Probiotic | 178 | 186 | 157 |
| Placebo | 147 | 127 | 256 |

One of the most important parameters to consider, and one that makes this initiative more innovative, is related to the weight variations in patients on chemotherapy. The volunteers who ingested the preparation with the DSM 22105 strain showed marked body weight recovery, increasing their weight by 2 to 6 kg, preferably 3 kg per month **(Table 9).** The weight difference between the beginning and after one month of treatment was statistically significant according to the t-test for paired data (p= 0.00492623). The ANOVA test for correlated samples shows that the weighted mean difference at the beginning, and at 30 and 60 days was significant p<0,03381 and Tukey's HSD test shows that the pair that indicated significant weighted mean difference was between 1 and 2 and between 1 and 3 (beginning, 30 days and 60 days).

**Table 9**

| TREATMENT | INITIAL WEIGHT Kg | WEIGHT 30 DAYS Kg | WEIGHT 60 DAYS Kg |
|---|---|---|---|
| PROBIOTIC (patient 1) | 68 | 72 | 72 |
| PROBIOTIC (patient 2) | 84 | 87 | 88 |
| PROBIOTIC (patient 3) | 67 | 70 | 71 |

**Table 10** presents the results of patients treated with the placebo, showing that the weighted mean difference between the beginning and end of treatment was not significant in these patients, thus confirming that the probiotic strain DSM 22105 benefits cancer patients by enabling them to gain weight.

**Table 10**

| TREATMENT | INITIAL WEIGHT Kg | WEIGHT 30 DAYS | WEIGHT 60 DAYS |
|---|---|---|---|
| PLACEBO (patient 4) | 71 | 72 | 70 |
| PLACEBO (patient 5) | 64 | 64 | 63 |

The microbiological recount in the stools of cancer volunteers (Figure 7) shows the lactic acid bacteria concentrations obtained from volunteers on chemotherapy, analyzed up to 60 days. At the beginning of the trial, all the volunteers presented a low concentration of basal lactobacilli from 10⁶ - 10⁷ CFU per gram of feces, which at 60 days had increased to levels around 10⁸ CFU per gram of feces. These data show that there is a clear recovery in the basal flora in the intestinal bacterial population

With regard to the product itself, the organoleptic properties of the ice cream -color, flavor and texture- remained the same during the monitoring process and were greatly appreciated by the participants. This reaffirms the fact that the strain incorporated into the ice cream does not change its biochemical properties and it also confirms the recovery of the sense of taste and the perception of flavor of cancer patients treated with the ice cream.

It is also important to consider the side effects of the consumption of the preparation. Not a single one of the patients in the two groups that ate the probiotic preparation had diarrhea after ingesting the product. Nor were there any other adverse reactions, such as mucositis, nausea or vomiting, like those described in most patients on chemotherapy and radiotherapy.

The preparation is conceptualized as a dessert. It was described as having a smooth texture and delicious flavor. The same appreciation was shown by both cancer volunteers and healthy volunteers. Even more importantly, for the cancer volunteers these three factors played a motivating role in maintaining regular consumption, despite the different states through which the body passed as a result of chemotherapy: an intense burning sensation in the upper digestive tract, general weakness, nausea, and constant headaches, as well as feelings of negativity. In addition, participants said that the probiotic ice cream was a nutritional supplement, since they did not stop eating it even on days when they were physically unwell. In fact, the ice cream was sometimes the only food they ate.

### APPLICATION EXAMPLES

### Example 1: Activation and evaluation of the probiotic strain

**1.1 Preparation of *DSM 22105.*** The strain was activated from a strain bank; after its activation, the bacteria were centrifuged, washed with physiological solution and adjusted to an optical density of OD₅₄₀ 0.6, then planted using torula on an MRS agar surface, and incubated for 24 hours at 25°C.

**1.2 Preparation of hospital pathogens and *Campylobacter. Jejuni.*** The enteropathogenic strains were activated and brought to a McFarland concentration of 0.5, then planted on a trypticase agar plate, in the case of the hospital pathogens, and on brucella agar with 5%horse blood, in the case of C. *jejuni.* 20 strains of *Shigella* spp., 20 strains of *Escherichia coli,* 30 strains of *Salmonella* spp., and 3 strains of C. *jejuni* isolated from patients with Diarrheic Syndrome were used.

For the inhibition trial conducted on trypticase agar plates for the development of the pathogenic strain, the lactic strain was inoculated on a microplate and incubation was carried out at 37° C for 12 and 24 hrs.

**1.3 Interaction trial with *Campylobacter Jejuni.*** This trial was conducted using the microplate interaction technique with modifications, plus Campbell's method. For the microplate interaction technique, the DSM 22105 strain was subjected to treatment using 5 conditions:
- Peroxide inhibition
- Lactic acid neutralization
- Peroxide inhibition and lactic acid neutralization
- Supernatant without bacteria
- Whole culture at an approximate concentration of 10⁹ CFU/ml.

For the Campbell's trial, pieces of MRS agar in which the lactic strain had already been grown were taken and they were deposited on the brucella agar surface that had been recently planted with *C*. *jejuni* strains; a disc with the lactic strain was placed in contact with the pathogen and another disc was placed with the surface in contact with the pathogenic strain. After the trial it was incubated in a microaerophilic environment at 37°C for 48 hours.

### Example 2: Culture and conditioning of the LPM O1 strain.

### 2.1 Preparation of culture medium.

*Hydrolysis of serum permeate:* The amount of enzyme needed (lactase or β-galactosidase) was added to serum permeate dissolved in distilled water and incubated for 1 to 4 hours at 40°C, under constant agitation.

*Addition of nutrients:* After hydrolysis had taken place, the remaining components from the culture medium were added, and it was adjusted to pH=6.0 and autoclaved for future use.

| **Culture medium components** | **Quantity (g/L)** |
|---|---|
| Lactoserum permeate | between 10 - 70; preferably between 30 - 50 |
| Enzyme (β-galactosidase) | between 0.1 - 4; preferably between 0.25 - 2 |
| Casein peptone | between 1 - 10; preferably between 4-7 |
| Yeast extract | between 1 - 8; preferably between 2-5 |
| Dihydrogen phosphate potassium | between 0.5 - 7; preferably between 1 - 4 |
| Polyoxyethylene (20) sorbitan monooleate | between 0.1 - 3; preferably between 0.5 - 1.5 |
| Magnesium sulfate | between 0.005 - 0.1; preferably between 0.01 - 0.03 |
| Manganese sulfate | between 0.05 - 2, preferably between 0.2 - 0.5 |

**2.2 Culture of the DSM 22105 strain.** After the DSM 22105 strain had been activated at 37°C, in consecutive picks, the first for 24 h and the second for 12 h, the concentration was adjusted to an OD of 0.5 (625 nm), in order to then inoculate the amount needed in the fermentation with the lactoserum permeated medium at 37°C, ensuring an initial concentration of bacteria of 10⁵ CFU/ml. This was incubated at 37°C for approximately 12 hours.

**2.3 Biomass harvesting.** Once the culture time was over, the broth was centrifuged with the biomass at 8000 rpm for 20 min at 15°C. After the sample was centrifuged, the supernatant was discarded and the decanted biomass stored for later use at 4°C.

**2.4 Mixture.** The biomass obtained from the culture was resuspended in whole milk (part of the ice cream composition) and the suspension was homogenized in an orbital agitator, to which was added the remaining liquid ice cream mixture (previously pasteurized). This was stored at 4°C, ready for the freezing stage of the probiotic ice cream.

### Example 3: Preparation of the dairy ice cream with a probiotic

### Ingredients

50 gr. ice cream base
40 gr. fat preparation
240 gr. sugar
1 It. milk
50 gr. dairy cream
1 gr. lactic bacteria with 10⁹ CFU
65 gr. flavoring

**3.1 Ingredient mixing:** A homogenous mixture of the different ingredients was obtained by heating the liquids (this is normally done using temperatures from 30 - 40°C) and then adding the powder base mixed with the stabilizer and emulsifier.

**3.2 Pasteurization:** A product free from viable pathogenic bacteria was obtained and the storage quality of the product was improved. Heating the mixture had the effect of dissolving the sugars, stabilizers and emulsifiers, while at the same time liquefying the fats. The recommended temperature for the continuous process is 80°C for 25 seconds and 72°C for 30 minutes for discontinuous pasteurization.

**3.3 Homogenization:** This stage is designed to obtain a fat globule of uniform size in emulsion, distribute the emulsifiers and milk proteins on the surface of the fat globule (protector colloids), improve the mixture and incorporate air, produce a smooth texture, and improve melting.

**3.4 Cooling:** Once the mixture was homogenized, it was cooled to 4°C to allow it to mature. This process can take from 2 to 4 hours.

**3.5 Incorporation of probiotic lactic bacteria:** Taking advantage of the coldness of the mixture, the lactic strain was incorporated by gentle mixing; making sure that a concentration of 10⁹ CFU/ml was uniform throughout the contents.

**3.6 Freezing:** The freezing and beating of the mixture transform it from a liquid state to a semi-solid state. The air content incorporated depends on the mixing speed in the freezer. The exit temperature from the freezer fluctuates around -5°C, and at this temperature practically 50% of the water in the mixture is in a solid state.

**3.7 Hardening and Storage:** Once the product comes out of the freezer in containers, it stabilizes rapidly, and most of the water that is still in liquid form freezes when the temperature is reduced to between -30°C and -40°C, which hardens the ice cream.
After the hardening process the ice cream can be stored in a freezer at -20°C until it is commercialized, at which point it is necessary to use appropriate cold channels in order to avoid undesirable changes in texture caused by fluctuations in storage temperature.
Finally, the ice cream must have a concentration of probiotic lactic bacteria of 10⁸ CFU/g.

**3.8 Stability of the product.** The specially prepared ice cream was produced in 200 ml. containers. From each container approximately 5 ml was extracted, homogenized and planted in different culture media for an evaluation of the viability (CFU/ml) and probiotic properties. The plates were incubated from 24 to 48 hours at 37° C. The ice cream was stored in a conventional freezer at -20° C.

### Example 4: Clinical trial.

**4.1 Inclusion Criterion.** Five volunteer cancer patients with colon, breast and ovarian cancer, among others, who were in chemotherapy cycles, were recruited, together with six healthy volunteers.

**4.2 Recruitment.** A randomized double blind trial was designed, in which, by drawing lots, the volunteers selected an orange or green preparation. The orange preparations contained only the ice cream and the green preparations contained the probiotic strain. This identification was only made at the moment of analyzing the results and the contents of the preparations were only known to the person in charge of the production process. The trial lasted 60 days and was preceded by a sensitization process, so that the volunteers began participation with informed consent before they drew lots for the preparation to be ingested.

**4.3 Dosification.** The clinical trial considered the consumption of the probiotic strain DSM 22105 at a probiotic concentration of 10⁷-10⁹ CFU/ml in each preparation, which was eaten for a month in a dose of 100 ml of ice cream daily. These preparations were stored in a freezer at -20°C. Each participant was given educational material with instructions to follow, including a chart on which to record symptoms.

**4.4 Monitoring of the preparations.** The following protocol was used to determine the viability of the preparation. Ice cream samples were planted serially to determine the number of colony forming units (CFU/ml of ice cream) on Rogosa and MRS agar plates. The results were analyzed after 48 hours of incubation at 37°C under microaerophilic conditions.

**4.5 Clinical evaluation.** At 0, 30 and 60 days, every participant was given a checkup, including a hemogram, and analyses of ESR, C-reactive protein, salivary IgA, the C3 factor of the compliment system, and of a stool sample. In addition, cancer

patients were asked to chart their diarrhea episodes on a daily basis, according to the classification provided by the World Health Organization (WHO):
*Grade 0* = No diarrhea.
*Grade 1* = Increase of 2 to 3 bowel movements/day over basal.
*Grade 2* = Increase of 4-6 bowel movements/day or nocturnal bowel movements, or moderate pains.
*Grade 3* = Increase of 7 - 9 bowel movements/day, incontinence or strong pains.
*Grade 4* = Increase of more than 10 bowel movements/day, visible bloody diarrhea or need for parenteral support.

**4.6 Stool analysis.** Three stool samples were taken from each participant, the first before the ice cream was ingested (T0), and then at 30 days (T30) and 60 days (T60) after consumption. The stool samples were transported in sterile containers in a cold chain. The samples were planted in MRS selective medium and incubated at 37°C under microaerophilic conditions (5% CO₂) for 24 - 48 hours. The methodology described made it possible to determine the presence and concentration of lactic bacteria present in volunteers' stools. Colonies with different morphology were selected from the plates with positive growth for incubation in MRS broth for 24 h at 37°C. Gram staining and a catalase test were carried out on the tubes with positive growth, and the catalase-negative and Gram-positive strains with bacillary or coccobacillary morphology were selected and kept in milk supplemented by yeast extract at -20°C, for molecular studies.

## Claims

1. Probiotic functional food suitable for patients on therapies such as chemotherapy and/or radiotherapy, **CHARACTERIZED in that** its formulation includes:
a) as active principle, a biomass of the viable probiotic strain that produces lactic acid, *Lactobacillus plantarum* strain DSM 22105, which is conditioned to withstand temperatures from -30 to -40ºC, and
b) cryopotectant agents.

2. Probiotic functional food according to Claim 1, **CHARACTERIZED in that** the biomass of DSM 22105 is found in a concentration range of 10⁶ - 10⁹ CFU/ml, preferably, 10⁸ CFU/ml, for at least 120 days.

3. Probiotic functional food according to Claim 1, **CHARACTERIZED in that** the cryoprotectant agents include:
a) lactoserum permeate; at a concentration of 10 - 70 g/L, preferably 30 - 50 g/L and,
b) sorbitan derivatives, preferably, polyoxyethylene (20) sorbitan monooleate, at a concentration of 0.1 - 3 g/L, preferably 0.5 - 1.5 g/L.

4. Probiotic functional food according to Claims 1 and 2, **CHARACTERIZED in that** said food includes jellies, desserts, juices and/or milk derivatives, preferably ice cream.

5. Probiotic functional food including the strain DSM 22105 for use in the inhibition of the growth in immunocompromised cancer patients of hospital pathogens, preferably *Salmonella enteritidis, Shigella* spp and *Campylobacter jejuni.*

6. Probiotic functional food including the strain DSM 22105 for use in the treatment of an individual on aggressive therapies, wherein the individual is immunocompromised, preferably, in cancer patients on chemotherapy and/or radiotherapy.

7. Probiotic functional food for the use according to Claim 6 for use in weight gain in cancer patients on radiotherapy and/or chemotherapy, between 2 and 6 kg, preferably, 3 kg per month.

8. Probiotic functional food for the use according to Claim 6 for use in counteracting the side effects produced by chemotherapy, preferably, mucositis, mouth dryness, and lesions and erosions in the digestive tract.

9. Probiotic functional food for the use according to Claim 6 for use in the recovery of the sense of taste and the perception of flavor in cancer patients on chemotherapy.

## Patentansprüche

1. Probiotisches funktionales Nahrungsmittel, geeignet für Patienten, welche Therapien wie z.B. einer Chemotherapie und/oder Radiotherapie unterzogen werden, **dadurch gekennzeichnet, dass** dessen Formulierung Folgendes umfasst:
a) als Wirkstoff, eine Biomasse von dem lebensfähigen probiotischen Stamm der Milchsäure produziert, *Lactobacillus plantarum,* Stamm DSM 22105, welcher dazu aufbereitet wurde, Temperaturen von -30 bis -40ºC auszuhalten, und
b) Kryoschutzmittel.

2. Probiotisches funktionales Nahrungsmittel nach Anspruch 1, **dadurch gekennzeichnet, dass** sich die Biomasse von DSM 22105 in einem Konzentrationsbereich von 10⁶-10⁹ CFU/ml, vorzugsweise 10⁸ CFU/ml, für mindestens 120 Tage befindet.

3. Probiotisches funktionales Nahrungsmittel nach Anspruch 1, **dadurch gekennzeichnet, dass** die Kryoschutzmittel Folgendes umfassen:
a) Laktoserumpermeat; bei einer Konzentration von 10-70 g/L, vorzugsweise 30 - 50 g/L und,
b) Sorbitanderivate, vorzugsweise Polyoxyethylen-(20)-Sorbitanmonooleat, bei einer Konzentration von 0,1-3 g/L, vorzugsweise 0,5 - 1,5 g/L.

4. Probiotisches funktionales Nahrungsmittel nach den Ansprüchen 1 und 2, **dadurch gekennzeichnet, dass** das genannte Nahrungsmittel Gelees, Nachtische, Säfte und/oder Milcherzeugnisse, vorzugsweise Eiscreme, umfasst.

5. Probiotisches funktionales Nahrungsmittel umfassend Stamm DSM 22105 für dessen Verwendung bei der Hemmung des Wachstums in immungeschwächten Krebspatienten von Krankenhauserregern, vorzugsweise *Salmonella enteritidis, Shigella* spp und *Campylobacter jejuni.*

6. Probiotisches funktionales Nahrungsmittel umfassend Stamm DSM 22105 für dessen Verwendung bei der Behandlung eines Individuums, das aggressiven Therapien unterzogen wird, wobei das Individuum immungeschwächt ist, vorzugsweise in Krebspatienten, die einer Chemotherapie und/oder Radiotherapie unterzogen werden.

7. Probiotisches funktionales Nahrungsmittel zur Verwendung nach Anspruch 6, für dessen Verwendung bei der Gewichtszunahme in Krebspatienten, die einer Radiotherapie und/oder Chemotherapie unterzogen werden, zwischen 2 und 6 kg, vorzugsweise, 3 kg pro Monat.

8. Probiotisches funktionales Nahrungsmittel zur Verwendung nach Anspruch 6, für dessen Verwendung bei der Entgegenwirkung der von der Chemotherapie hervorgerufenen Nebenwirkungen, vorzugsweise Mukositis, Mundtrockenheit, und Verletzungen und Erosionen im Verdauungstrakt.

9. Probiotisches funktionales Nahrungsmittel zur Verwendung nach Anspruch 6, für dessen Verwendung bei der Rückgewinnung des Geschmackssinnes und der Empfindung von Geschmacksrichtung in Krebspatienten, die einer Chemotherapie unterzogen werden.

## Revendications

1. Aliment fonctionnel probiotique convenant pour des patients traités par des thérapies telles que chimiothérapie et/ou radiothérapie, **caractérisé en ce que** sa formulation comporte :
a) comme principe actif, une biomasse de la souche probiotique variable qui produit de l'acide lactique, *Lactobacillus plantarum,* souche DSM 2215, qui est conditionné pour supporter des températures de -30 à -40ºC, et
b) des agents cryoprotecteurs.

2. Aliment fonctionnel probiotique selon la revendication 1, **caractérisé en ce que** la biomasse de DSM 22105 se trouve dans un intervalle de concentration de 10⁶ - 10⁹ UFC/ml, de préférence, 10⁸ UFC/ml, pendant au moins 120 jours.

3. Aliment fonctionnel probiotique selon la revendication 1, **caractérisé en ce que** les agents cryoprotecteurs comportent :
a) du perméat de lactosérum ; à une concentration de 10 - 70 g/l, de préférence 30 - 50 g/l et,
b) des dérivés de sorbitane, de préférence du monooléate de polyoxyéthylène (20) sorbitane, à une concentration de 0,1 - 3 g/l, de préférence 0,5 - 1,5 g/l.

4. Aliment fonctionnel probiotique selon les revendications 1 et 2, **caractérisé en ce que** ledit aliment inclut des gelées, des desserts, des jus et/ou des dérivés du lait, de préférence des glaces.

5. Aliment fonctionnel probiotique comportant la souche DSM 22105 pour son utilisation dans l'inhibition de la croissance chez des patients immunodéprimés atteints d'un cancer de pathogènes hospitaliers, de préférence de *Salmonella enteritidis, Shigella* spp et *de Campylobacter jejuni.*

6. Aliment fonctionnel probiotique comportant la souche DSM 22105 pour son utilisation dans le traitement d'un individu traité par des thérapies agressives, dans lequel l'individu est immunodéprimé, de préférence, chez des patients atteints d'un cancer traités par chimiothérapie et/ou radiothérapie.

7. Aliment fonctionnel probiotique pour son utilisation selon la revendication 6, pour son utilisation dans la prise de poids chez des patients atteints d'un cancer traités par radiothérapie et/ou chimiothérapie, entre 2 et 6 kg, de préférence, 3 kg par mois.

8. Aliment fonctionnel probiotique pour son utilisation selon la revendication 6, pour son utilisation pour compenser les effets secondaires produits par la chimiothérapie, de préférence la mucite, la sécheresse buccale, et des lésions et érosions dans le tube digestif.

9. Aliment fonctionnel probiotique pour son utilisation selon la revendication 6, pour son utilisation dans la récupération du sens du goût et la perception de la saveur chez des patients atteints d'un cancer traités par chimiothérapie.
